# EUROPEAN PATENT APPLICATION

(11) **EP 2 939 998 A1**
(43) Date of publication of application: **04.11.2015**
(21) Application number: 13867283.7
(22) Date of filing: 26.12.2013
(51) Int. Cl.: C07C 43/04, C10M 105/18, C10M 107/02, C10N 30/02, C10N 40/02, C10N 40/04, C10N 40/06, C10N 40/08, C10N 40/25, C10N 40/30

(54) **DIALKYL ETHER, AND BASE OIL FOR LUBRICANT OILS AND LUBRICANT OIL COMPOSITION EACH CONTAINING SAME**

(30) Priority: 27.12.2012 JP 2012285617
(71) Applicant: Idemitsu Kosan Co., Ltd, Tokyo 100-8321 (JP)
(72) Inventor: MONOI, Miki, Sodegaura-shi Chiba 299-0293 (JP); YOSHIDA, Yukio, Sodegaura-shi Chiba 299-0293 (JP)
(74) Representative: Hoffmann Eitle
(86) International application number: PCT/JP2013/084829
(87) International publication number: WO 2014/104174

(57) **Abstract**

The present invention provides a dialkyl ether represented by the following formula (1), and a lubricating base oil and a lubricating oil composition containing the dialkyl ether; the lubricating base oil and the lubricating oil composition exhibit low viscosity, excellent viscosity-temperature characteristics, and excellent low-temperature fluidity. 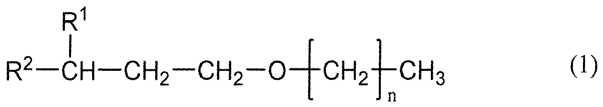 (wherein each of R¹ and R² represents a C1 to C20 alkyl group, and n is an integer of 1 to 20).

## Description

### TECHNICAL FIELD

The present invention relates to a dialkyl ether, and to a lubricating base oil and a lubricating oil composition containing the dialkyl ether.

### BACKGROUND ART

In recent years, CO₂ emission has been required to be reduced more and more from the viewpoint of protection of the global environment. Under such circumstances, there is demand for a drastic improvement in fuel-saving performance of lubricating oil. For attaining fuel savings, reduction of viscous resistance is an effective measure, and a variety of low-viscosity lubricating base oils have been developed. Hitherto, (1) ester, (2) poly-α-olefin, (3) silicone oil, and the like have been developed as low-viscosity lubricating base oils. However, these lubricating base oils have the following problems.

Specifically, when used in an engine oil, ester (1), having high polarity, adversely affects organic parts, resulting in swelling of rubber sealing parts. Poly-α-olefin (2) does not affect organic parts, but difficulty is encountered in establishing suitable balance between viscosity index and pour point (low-temperature flowability), when the viscosity thereof is reduced. Silicone oil (3) has low viscosity and high viscosity index, and exhibits excellent low-temperature flowability. However, silicon oil has problems including poor lubricity and poor compatibility with existing additives.

In order to solve these problems, there has recently been developed a lubricating base oil which contains a compound having an ether bond and which exhibits a kinematic viscosity at 40°C and an aniline point falling within specific ranges (see Patent Document 1).

It is true that a lubricating base oil containing a compound having an ether bond has low viscosity and high viscosity index, exhibits excellent low-temperature flowability and is highly compatible to organic parts, thereby enabling use in a lubricating oil such as an engine oil for an internal combustion engine or the like. However, in order to meet needs for further improved fuel-saving performance, such a lubricating base oil must attain a further reduced viscosity and well-balanced viscosity-temperature characteristics and low-temperature flowability.

Specifically, the lubricating base oil disclosed in Patent Document 1 exhibits a kinematic viscosity, as measured at 100°C, of 3.2 to 2.4 mm²/s. In this case, the lower the viscosity, the lower the viscosity index. Currently, there has not been clearly elucidated the structure of the ether compound which enables to attain well-balanced viscosity index and low-temperature flowability of the base oil when the viscosity thereof is further reduced. Patent Documents 2 and 3 disclose a lubricating base oil containing a monoether. However, the disclosed alkyl group combinations at both ends are limited. Patent Documents 4 to 7 and Non-Patent Document 1 disclose monoether compounds, but do not disclose a monoether that can improve viscosity index and low-temperature flowability or a lubricating base oil containing the monoether.
Patent Document 1: WO 2004/058928 A1
Patent Document 2: WO 2004/090082 A1
Patent Document 3: JP 2005-344017 A
Patent Document 4: JP 49-12746 B
Patent Document 5: JP 48-039507 A
Patent Document 6: JP 57-117596 A
Patent Document 7: JP 09-087223 A

Non-Patent Document 1: Wilhelm Schlenk Jr., "Konfigurativ Stetige Gitterzuordnung der Gastmolekule," Liebigs Ann. Chem. 1973, 1156-1178

### SUMMARY OF INVENTION

### TECHNICAL PROBLEM

The present invention has been conceived in view of the foregoing. Thus, an object of the present invention is to provide an ether which exhibits low viscosity, excellent viscosity-temperature characteristics and excellent low-temperature fluidity. Another object is to provide a lubricating base oil and a lubricating oil composition containing the ether.

### SOLUTION TO PROBLEM

The present inventors have conducted extensive studies to attain the objects, and have found that the objects can be attained by a dialkyl ether having a specific branch position and a specific combination of end alkyl groups.

That is, the present invention provides the following.
1. A dialkyl ether represented by the following formula (1): 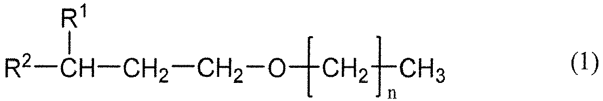 (wherein each of R¹ and R² represents a C1 to C20 alkyl group, and n is an integer of 1 to 20).
2. The dialkyl ether according to the item 1, wherein R² in the formula (1) is a C3 to C10 branched alkyl group.
3. The dialkyl ether according to the item 2, wherein R² in the formula (1) is a 2,2-dimethylpropyl group or a 4-methylpentyl group.
4. The dialkyl ether according to any one of the items 1 to 3, wherein R¹ in the formula (1) is a C1 to C3 linear-chain alkyl group.
5. The dialkyl ether according to the item 4, wherein R¹ in the formula (1) is a methyl group.
6. The dialkyl ether according to any one of the items 1 to 5, wherein n in the formula (1) is an integer of 7 to 20.
7. The dialkyl ether according to the item 6, wherein n in the formula (1) is an integer of 7 to 12.
8. The dialkyl ether according to the item 7, wherein n in the formula (1) is an integer of 9 to 11.
9. The dialkyl ether according to any one of the items 1 to 8, which has 30 or less carbon atoms in total.
10. The dialkyl ether according to any one of the items 1 to 9, which has an aniline point of 40°C or higher.
11. The dialkyl ether according to any one of the items 1 to 10, which has a flash point of 140°C or higher.
12. The dialkyl ether according to any one of the items 1 to 11, which has a pour point of 0°C or lower.
13. A lubricating base oil comprising at least one of dialkyl ether represented by the following formula (1): 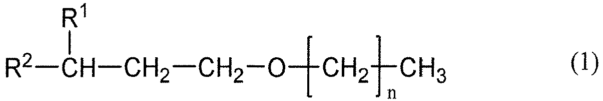 (wherein each of R¹ and R² represents a C1 to C20 alkyl group, and n is an integer of 1 to 20).
14. The lubricating base oil according to the item 13, wherein a kinematic viscosity at 100°C is 5.0 mm²/s or less, and a viscosity index is 150 or more.
15. The lubricating base oil according to the items 13 or 14, wherein a pour point is 0°C or lower.
16. The lubricating base oil according to the item 15, wherein a pour point is -25°C or lower.
17. The lubricating base oil according to any one of the items 13 to 16, which further contains a hydrocarbon compound.
18. The lubricating base oil according to the item 17, wherein the hydrocarbon compound is a poly-α-olefin.
19. A lubricating oil composition comprising the lubricating base oil according to any one of the items 13 to 18, and an additive.
20. The lubricating base oil according to any one of the items 13 to 18, which is for use in a drive system.
21. The lubricating base oil according to any one of the items 13 to 18, which is for use in an internal combustion engine.

### ADVANTAGEOUS EFFECTS OF INVENTION

The present invention enables provision of an ether which exhibits low viscosity, excellent viscosity-temperature characteristics and excellent low-temperature fluidity, and a lubricating base oil and a lubricating oil composition containing the ether.

### DESCRIPTION OF EMBODIMENTS

### [Dialkyl ether]

The dialkyl ethers falling within the scope of the present invention are represented by the following formula (1): 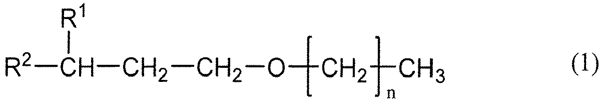 (wherein each of R¹ and R² represents a C1 to C20 alkyl group, and n is an integer of 1 to 20).

Examples of the alkyl group of R¹ and R² in the formula (1) include linear-chain and branched alkyl groups.

Examples of the linear-chain alkyl group include methyl, ethyl, n-propyl, n-butyl, n-pentyl, n-hexyl, n-heptyl, n-octyl, n-nonyl and n-decyl.

Specific examples of the branched alkyl group include isopropyl, 1-methylpropyl, 2-methylpropyl, t-butyl, 1,1-dimethylpropyl, 2,2-dimethylpropyl, 1,2-dimethylpropyl, 1-ethylpropyl, 2-ethylpropyl, 1,1-diethylpropyl, 1-methylbutyl, 2-methylbutyl, 3-methylbutyl, 1,1-dimethylbutyl, 2,2-dimethylbutyl, 3,3-dimethylbutyl, 1,3,3-trimethylbutyl, 1-ethylbutyl, 2-ethylbutyl, 3,3-dimethylbutyl, 1-propylbutyl, 1-methylpentyl, 2-methylpentyl, 3-methylpentyl, 4-methylpentyl, 4,4-dimethylpentyl, 1-ethylpentyl, 2-ethylpentyl, 3-ethylpentyl, 4-ethylpentyl, 1-propylpentyl, 2-propylpentyl, 1-butylpentyl, 1-methylhexyl, 2-methylhexyl, 3-methylhexyl, 4-methylhexyl, 5-methylhexyl, 5,5-dimethylhexyl, 1-ethylhexyl, 2-ethylhexyl, 3-ethylhexyl, 4-ethylhexyl, 1-propylhexyl, 2-propylhexyl, 3-propylhexyl, 1-butylhexyl, 2-butylhexyl, 1-methylheptyl, 2-methylheptyl, 3-methylheptyl, 4-methylheptyl, 5-methylheptyl, 6-methylheptyl, 6,6-dimethylheptyl, 1-ethylheptyl, 2-ethylheptyl, 3-ethylheptyl, 4-ethylheptyl, 5-ethylheptyl, 1-propylheptyl, 2-propylheptyl, 3-propylheptyl, 1-methyloctyl, 2-methyloctyl, 3-methyloctyl, 4-methyloctyl, 5-methyloctyl, 6-methyloctyl, 7-methyloctyl, 7,7-dimethyloctyl, 1-ethyloctyl, 2-ethyloctyl, 3-ethyloctyl, 4-ethyloctyl, 5-ethyloctyl, 6-ethyloctyl, 1-methylnonyl, 2-methylnonyl, 3-methylnonyl, 4-methylnonyl, 5-methylnonyl, 6-methylnonyl, 7-methylnonyl, 8-methylnonyl, and 3,5,5-trimethylhexyl.

R¹ is preferably a C1 to C3 linear-chain alkyl group, and R² is preferably a C3 to C10 branched alkyl group, more specifically, a 2,2-dimethylpropyl group or a 4-methylpentyl group.

The "n" in the formula (1) is preferably an integer of 7 to 20, more preferably 8 to 16, still more preferably 7 to 12, most preferably 9 to 11.

The dialkyl ether represented by the formula (1) preferably has 30 or less carbon atoms in total in the molecule thereof, more preferably 10 to 25 carbon atoms.

The dialkyl ether of the present invention preferably has an aniline point of 40°C or higher, more preferably 45 to 90°C. In the case where the aniline point of the dialkyl ether is 40°C or higher, a sealing part made of plastic material, rubber, or the like does not swell when in contact with the lubricating base oil containing the dialkyl ether. In the case where the aniline point is 90°C or lower, contraction of such a sealing part is prevented.

The dialkyl ether of the present invention preferably has a flash point of 140°C or higher, more preferably 150°C or higher. In the case where the flash point is 140°C or higher, evaporation of the lubricating base oil containing the dialkyl ether of the present invention is suppressed, thereby prolonging the life of the lubricating oil.

When the kinematic viscosity, as measured at 100°C, is P mm²/s, and the kinematic viscosity, as measured at 40°C, is Q mm²/s, the dialkyl ether of the present invention preferably satisfies the relationship (a) a P of 2.0 mm²/s or more, and a viscosity index of 130 or more, or the relationship (b) a P of less than 2.0 mm²/s, and Q ≤ 4.478 x P - 2.9234.

The dialkyl ether of the present invention preferably has a kinematic viscosity, as measured at 40°C, of 5.8 mm²/s or less, and a kinematic viscosity, as measured at 100°C, of 2.1 mm²/s or less. In addition, the dialkyl ether of the present invention preferably has a pour point of 0°C or lower, more preferably -30°C or lower.

### [Lubricating base oil]

The lubricating base oil of the present invention contains at least one of the above dialkyl ethers. Depending on the isolation technique, the lubricating base oil may further contain unreacted raw material and by-products.

The amount of dialkyl ether, with respect to the entire amount of the lubricating base oil of the present invention, is preferably 50 to 100 mass%, more preferably 70 to 100 mass%, still more preferably 80 to 100 mass%, particularly preferably 90 to 100 mass%.

The lubricating base oil of the present invention may further contain, in addition to the above dialkyl ether, another base oil such as a mineral oil or a hydrocarbon compound.

Examples of the hydrocarbon compound include poly-α-olefin, ethylene-propylene copolymer, esters (monoester, diester, polyester, etc.), polyethers (e.g., polyalkylene glycol), and alkylbenzene. Among them, a high-viscosity poly-α-olefin; specifically, a poly-α-olefin having a kinematic viscosity, as measured at 100°C, of 100 mm²/s or more is preferred, and the kinematic viscosity is more preferably 120 mm²/s or more, still more preferably 140 mm²/s or more. From a view point of low-temperature flowability, preferably, the pour point is -40°C or lower, and the viscosity index is 170 or more.

The lubricating base oil of the present invention preferably has a kinematic viscosity, as measured at 100°C, of 5.0 mm²/s or less, more preferably 4.5 mm²/s or less, still more preferably 3.0 mm²/s or less, particularly preferably 2.7 mm²/s or less, most preferably 2.5 mm²/s or less.

The lubricating base oil of the present invention preferably has a viscosity index of 150 or more, more preferably 170 or more, still more preferably 190 or more.

The lubricating base oil of the present invention preferably has a pour point of 0°C or lower, more preferably -25°C or lower, still more preferably -30°C or lower.

When the kinematic viscosity as measured at 100°C, viscosity index, and pour point fall within the above ranges, low viscosity, low evaporation performance, and energy- and fuel-saving performance can be attained.

### EXAMPLES

The present invention will next be described in more detail by way of examples, which should not be construed as limiting the invention thereto.

### Example 1

### Synthesis of 1-(3,7-dimethyloctoxy)decane

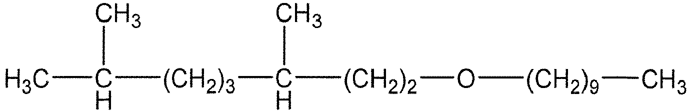

To a 2-L glass flask, 158 g of 3,7-dimethyl-1-octanol, 221 g of 1-bromodecane, 32 g of tetrabutylammonium bromide, and 454 g of aqueous sodium hydroxide (236 g of sodium hydroxide dissolved in 218 g of water) were added, and the mixture was allowed to react under stirring at 70°C for 5 hours.

After completion of reaction, the reaction mixture was transferred to a separating funnel, and the aqueous layer was removed. The remaining organic layer was washed five times with 500 mL of water. The washed organic layer was distilled under reduced pressure, to thereby yield 208 g of 1-(3,7-dimethyloctoxy)decane.

Then, the kinematic viscosity, viscosity index, pour point, aniline point, and flash point of the thus-yielded 1-(3,7-dimethyloctoxy)decane were measured through the following methods. Table 1 shows the results.

### (1) Kinematic viscosity

Measured in accordance with the method described in JIS K2283.

### (2) Viscosity index

Measured in accordance with the method described in JIS K2283.

### (3) Pour point

Measured in accordance with the method described in JIS K2269.

### (4) Aniline point

Measured in accordance with the method described in JIS K2256.

### (5) Flash point

Measured in accordance with the method described in JIS K2265.

### Example 2

### Synthesis of 1-(3,7-dimethyloctoxy)dodecane

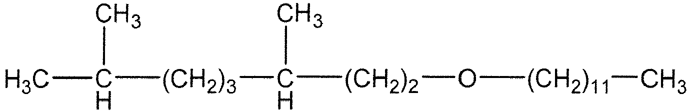

The procedure of Example 1 including reaction and posttreatment was conducted, except that 249 g of 1-bromododecane was used instead of 221 g of 1-bromodecane, to thereby yield 228 g of 1-(3,7-dimethyloctoxy)dodecane. The kinematic viscosity, viscosity index, pour point, aniline point, and flash point of the thus-yielded compound were measured in the same manner as employed in Example 1. Table 1 shows the results.

### Example 3

### Synthesis of 1-(3,7-dimethyloctoxy)undecane

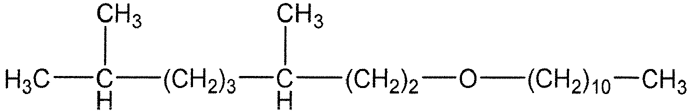

The procedure of Example 1 including reaction and posttreatment was conducted, except that 235 g of 1-bromoundecane was used instead of 221 g of 1-bromodecane, to thereby yield 190 g of 1-(3,7-dimethyloctoxy)undecane. The kinematic viscosity, viscosity index, pour point, aniline point, and flash point of the thus-yielded compound were measured in the same manner as employed in Example 1. Table 1 shows the results.

### Example 4

### Synthesis of 1-(3,5,5-trimethylhexoxy)dodecane

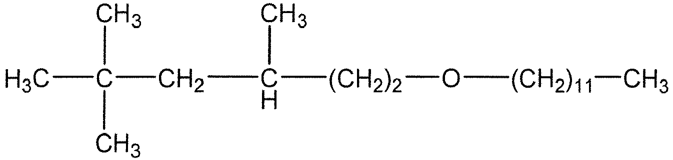

The procedure of Example 1 including reaction and posttreatment was conducted, except that 144 g of 3,5,5-trimethyl-1-hexanol and 249 g of 1-bromododecane were used instead of 158 g of 3,7-dimethyl-1-octanol and 221 g of 1-bromodecane, to thereby yield 225 g of 1-(3,5,5-trimethylhexoxy)dodecane. The kinematic viscosity, viscosity index, pour point, aniline point, and flash point of the thus-yielded compound were measured in the same manner as employed in Example 1. Table 1 shows the results.

### Example 5

### Synthesis of 1-(3,5,5-trimethylhexoxy)decane

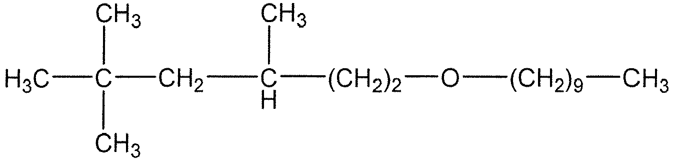

The procedure of Example 1 including reaction and posttreatment was conducted, except that 144 g of 3,5,5-trimethyl-1-hexanol was used instead of 158 g of 3,7-dimethyl-1-octanol, to thereby yield 200 g of 1-(3,5,5-trimethylhexoxy)decane. The kinematic viscosity, viscosity index, pour point, aniline point, and flash point of the thus-yielded compound were measured in the same manner as employed in Example 1. Table 1 shows the results.

### Example 6

### Synthesis of 1-(3,5,5-trimethylhexoxy)undecane

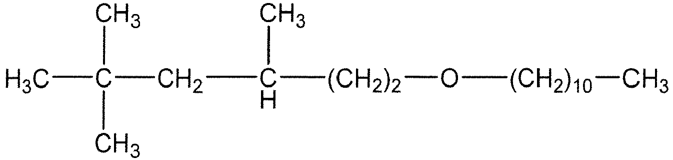

The procedure of Example 1 including reaction and posttreatment was conducted, except that 144 g of 3,5,5-trimethyl-1-hexanol and 235 g of 1-bromoundecane were used instead of 158 g of 3,7-dimethyl-1-octanol and 221 g of 1-bromodecane, to thereby yield 195 g of 1-(3,5,5-trimethylhexoxy)undecane. The kinematic viscosity, viscosity index, pour point, aniline point, and flash point of the thus-yielded compound were measured in the same manner as employed in Example 1. Table 1 shows the results.

### Example 7

### Synthesis of 1-(3,5,5-trimethylhexoxy)tetradecane

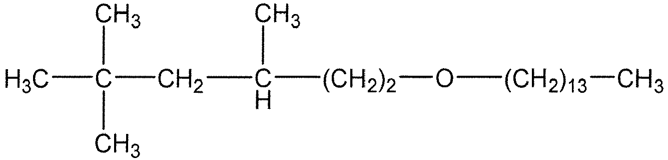

The procedure of Example 1 including reaction and posttreatment was conducted, except that 144 g of 3,5,5-trimethyl-1-hexanol and 277 g of 1-bromotetradecane were used instead of 158 g of 3,7-dimethyl-1-octanol and 221 g of 1-bromodecane, to thereby yield 248 g of 1-(3,5,5-trimethylhexoxy)tetradecane. The kinematic viscosity, viscosity index, pour point, aniline point, and flash point of the thus-yielded compound were measured in the same manner as employed in Example 1. Table 1 shows the results.

### Comparative Example 1

### Synthesis of 7-(octoxymethyl)pentadecane

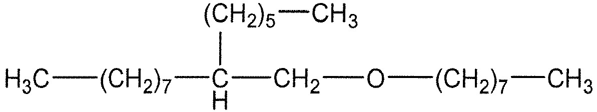

The procedure of Example 1 including reaction and posttreatment was conducted, except that 242 g of 2-hexyl-1-decanol and 193 g of 1-bromooctane were used instead of 158 g of 3,7-dimethyl-1-octanol and 221 g of 1-bromodecane, to thereby yield 230 g of 7-(octoxymethyl)pentadecane. The kinematic viscosity, viscosity index, pour point, aniline point, and flash point of the thus-yielded compound were measured in the same manner as employed in Example 1. Table 1 shows the results.

### Comparative Example 2

### Evaluation of 7-(decoxymethyl)pentadecane

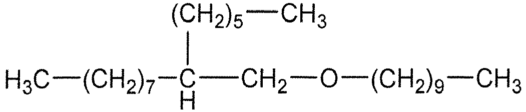

The kinematic viscosity, viscosity index, pour point, aniline point, and flash point of 7-(decoxymethyl)pentadecane, disclosed in Japanese Patent No. 4769463, were measured in the same manner as employed in Example 1. Table 1 shows the results.

### Comparative Example 3

### Evaluation of 1,3-dimethylbutyl dodecyl ether

The kinematic viscosity, viscosity index, pour point, aniline point, and flash point of 1,3-dimethylbutyl dodecyl ether, used in Examples 1 and 2 of Patent Document 6, were measured in the same manner as employed in Example 1. Table 1 shows the results.

### Comparative Example 4

### Evaluation of 1,3-dimethylbutyl tetradecyl ether

The kinematic viscosity, viscosity index, pour point, aniline point, and flash point of 1,3-dimethylbutyl tetradecyl ether, used in Examples 3 to 7 of Patent Document 6, were measured in the same manner as employed in Example 1. Table 1 shows the results.

### Comparative Example 5

### Evaluation of 1-methylpropyl tetradecyl ether

The kinematic viscosity, viscosity index, pour point, aniline point, and flash point of 1-methylpropyl tetradecyl ether, used in Examples 12 and 13 of Patent Document 6, were measured in the same manner as employed in Example 1. Table 1 shows the results.

### Comparative Example 6

### Evaluation of 1-methylpropyl octadecyl ether

The kinematic viscosity, viscosity index, pour point, aniline point, and flash point of 1-methylpropyl octadecyl ether, used in Example 14 of Patent Document 6, were measured in the same manner as employed in Example 1. Table 1 shows the results.

[Table 1]

**Table 1**

| | Kinematic viscosity (mm²/s) | | Viscosity index | Pour point | Aniline point | Flash point | Density |
|---|---|---|---|---|---|---|---|
| | 40°C | 100°C | | (°C) | (°C) | (°C) | (g/cm³) |
| Example 1 | 4.791 | 1.736 | - | -37.5 | 56.2 | 174 | 0.8172 |
| Example 2 | 6.297 | 2.121 | 154 | -22.5 | 65.1 | 192 | 0.8191 |
| Example 3 | 5.524 | 1.927 | - | -30 | 60.7 | 182 | 0.8186 |
| Example 4 | 5.745 | 2.02 | 169 | -27.5 | 61.8 | 182 | 0.8192 |
| Example 5 | 4.289 | 1.629 | - | -40 | 52 | 160 | 0.8164 |
| Example 6 | 4.979 | 1.813 | - | -35 | 57.2 | 170 | 0.8164 |
| Example 7 | 7.468 | 2.442 | 170 | -15 | 69.8 | 196 | 0.8211 |
| Comparative Example 1 | 6.808 | 2.135 | 117 | ≤-50 | 79.5 | 202 | 0.8234 |
| Comparative Example 2 | 8.372 | 2.494 | 129 | ≤-45 | 86 | 211 | 0.8283 |
| Comparative Example 3 | 3.401 | 1.373 | - | -25 | 45.9 | 157 | 0.8098 |
| Comparative Example 4 | 4.531 | 1.706 | - | -15 | 55.7 | 174 | 0.8131 |
| Comparative Example 5 | 3.779 | 1.502 | - | -10 | 44.5 | 161 | 0.8122 |
| Comparative Example 6 | 6.658 | 2.298 | 187 | 20 | 64 | 195 | 0.8207 |

### Example 8

The compound obtained in Example 1 and poly-α-olefin (kinematic viscosity at 100°C: 150 mm²/s) were mixed at a mass ratio of 74:26. The kinematic viscosity, viscosity index, and pour point of the thus-obtained mixture were measured in the same manner as employed in Example 1. Table 2 shows the results.

### Example 9

The compound obtained in Example 2 and poly-α-olefin used in Example 8 were mixed at a mass ratio of 78:22. The kinematic viscosity, viscosity index, and pour point of the thus-obtained mixture were measured in the same manner as employed in Example 1. Table 2 shows the results.

### Example 10

The compound obtained in Example 3 and poly-α-olefin used in Example 8 were mixed at a mass ratio of 76:24. The kinematic viscosity, viscosity index, and pour point of the thus-obtained mixture were measured in the same manner as employed in Example 1. Table 2 shows the results.

### Example 11

The compound obtained in Example 4 and poly-α-olefin used in Example 8 were mixed at a mass ratio of 77:23. The kinematic viscosity, viscosity index, and pour point of the thus-obtained mixture were measured in the same manner as employed in Example 1. Table 2 shows the results.

### Example 12

The compound obtained in Example 5 and poly-α-olefin used in Example 8 were mixed at a mass ratio of 73:27. The kinematic viscosity, viscosity index, and pour point of the thus-obtained mixture were measured in the same manner as employed in Example 1. Table 2 shows the results.

### Comparative Example 7

The compound obtained in Comparative Example 3 and poly-α-olefin used in Example 8 were mixed at a mass ratio of 69:31. The kinematic viscosity, viscosity index, and pour point of the thus-obtained mixture were measured in the same manner as employed in Example 1. Table 2 shows the results.

### Comparative Example 8

The compound obtained in Comparative Example 4 and poly-α-olefin used in Example 8 were mixed at a mass ratio of 73:27. The kinematic viscosity, viscosity index, and pour point of the thus-obtained mixture were measured in the same manner as employed in Example 1. Table 2 shows the results.

### Comparative Example 9

The compound obtained in Example 5 and poly-α-olefin used in Example 8 were mixed at a mass ratio of 70:30. The kinematic viscosity, viscosity index, and pour point of the thus-obtained mixture were measured in the same manner as employed in Example 1. Table 2 shows the results.

[Table 2]

**Table 2**

| | Dialkyl ether | | Poly-α-olefin | Kinematic viscosity (mm²/s) | | Viscosity index | Pour point |
|---|---|---|---|---|---|---|---|
| | Compound | Content (mass%) | Content (mass%) | 40°C | 100°C | | (°C) |
| Example 8 | Example 1 | 74 | 26 | 15.675 | 4.456 | 220 | ≤-40 |
| Example 9 | Example 2 | 78 | 22 | 16.562 | 4.537 | 208 | -25 |
| Example 10 | Example 3 | 76 | 24 | 15.725 | 4.488 | 223 | -30 |
| Example 11 | Example 4 | 77 | 23 | 15.787 | 4.507 | 223 | -27.5 |
| Example 12 | Example 5 | 73 | 27 | 15.339 | 4.493 | 233 | ≤-40 |
| Comparative Example 7 | Comparative Example 3 | 69 | 31 | 15.042 | 4.549 | 249 | -15 |
| Comparative Example 8 | Comparative Example 4 | 73 | 27 | 15.599 | 4.518 | 230 | -20 |
| Comparative Example 9 | Comparative Example 5 | 70 | 30 | 15.3 | 4.587 | 247 | -10 |

As is clear from comparison of Examples 1 to 7 with Comparative Examples 1 to 6 shown in Table 1, and from comparison of Examples 8 to 12 with Comparative Examples 7 to 9 shown in Table 2, the dialkyl ethers falling within the scope of the present invention exhibit low viscosity, excellent viscosity-temperature characteristics, and excellent low-temperature fluidity, which are required of a base oil of a lubricating oil composition.

### INDUSTRIAL APPLICABILITY

The dialkyl ether of the present invention exhibits low viscosity, excellent viscosity-temperature characteristics, and excellent low-temperature fluidity. Thus, the ether is suitably used in a drive system, an internal combustion engine, or the like.

## Claims

1. A dialkyl ether represented by the following formula (1): 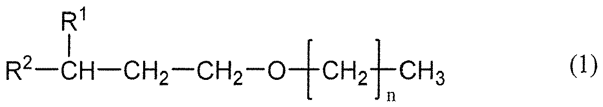 (wherein each of R¹ and R² represents a C1 to C20 alkyl group, and n is an integer of 1 to 20).

2. The dialkyl ether according to claim 1, wherein R² in the formula (1) is a C3 to C10 branched alkyl group.

3. The dialkyl ether according to claim 2, wherein R² in the formula (1) is a 2,2-dimethylpropyl group or a 4-methylpentyl group.

4. The dialkyl ether according to any one of claims 1 to 3, wherein R¹ in the formula (1) is a C1 to C3 linear-chain alkyl group.

5. The dialkyl ether according to claim 4, wherein R¹ in the formula (1) is a methyl group.

6. The dialkyl ether according to any one of claims 1 to 5, wherein n in the formula (1) is an integer of 7 to 20.

7. The dialkyl ether according to claim 6, wherein n in the formula (1) is an integer of 7 to 12.

8. The dialkyl ether according to claim 7, wherein n in the formula (1) is an integer of 9 to 11.

9. The dialkyl ether according to any one of claims 1 to 8, which has 30 or less carbon atoms in total.

10. The dialkyl ether according to any one of claims 1 to 9, which has an aniline point of 40°C or higher.

11. The dialkyl ether according to any one of claims 1 to 10, which has a flash point of 140°C or higher.

12. The dialkyl ether according to any one of claims 1 to 11, which has a pour point of 0°C or lower.

13. A lubricating base oil comprising at least one of dialkyl ether represented by the following formula (1): 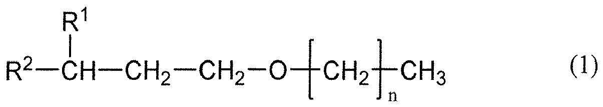 (wherein each of R¹ and R² represents a C1 to C20 alkyl group, and n is an integer of 1 to 20).

14. The lubricating base oil according to claim 13, wherein a kinematic viscosity at 100°C is 5.0 mm²/s or less, and a viscosity index is 150 or more.

15. The lubricating base oil according to the claims 13 or 14, wherein a pour point is 0°C or lower.

16. The lubricating base oil according to claim 15, wherein a pour point is -25°C or lower.

17. The lubricating base oil according to any one of claims 13 to 16, which further comprises a hydrocarbon compound.

18. The lubricating base oil according to claim 17, wherein the hydrocarbon compound is a poly-α-olefin.

19. A lubricating oil composition comprising the lubricating base oil according to any one of claims 13 to 18, and an additive.

20. The lubricating base oil according to any one of claims 13 to 18, which is for use in a drive system.

21. The lubricating base oil according to any one of claims 13 to 18, which is for use in an internal combustion engine.
